# EUROPEAN PATENT SPECIFICATION

(11) **EP 0 572 494 B1**
(45) Date of publication and mention of the grant of the patent: **25.08.1999**
(21) Application number: 92905485.6
(22) Date of filing: 18.02.1992
(51) Int. Cl.: A61K 9/10, A61K 9/06, A61K 31/375, A61K 31/19, A61K 31/40

(54) **COMPOSITION FOR USE IN TRANSDERMAL ADMINISTRATION**
ZUSAMMENSETZUNG ZUR VERWENDUNG IN DER TRANSDERMALEN VERABREICHUNG
COMPOSITION D'ADMINISTRATION TRANSDERMIQUE

(30) Priority: 18.02.1991 AU 465191; 19.08.1991 AU 784691; 19.08.1991 AU 784791; 19.08.1991 AU 784891; 21.11.1991 US 795499
(43) Date of publication of application: 08.12.1993
(73) Proprietor: COMMONWEALTH SCIENTIFIC AND INDUSTRIAL RESEARCH ORGANISATION, Campbell, ACT 2612 (AU)
(72) Inventor: TAYLOR, Reginald, Morton, Hawthorn, SA 5062 (AU); WILSON, David, John, Balmain, NSW 2041 (AU)
(74) Representative: West, Alan Harry
(86) International application number: AU9200058
(87) International publication number: WO9214442

(56) References cited:
- EP-A- 0 151 953
- EP-A- 0 156 080
- EP-A- 0 271 332
- EP-A- 0 272 045
- WO-A-92/08445
- FR-A- 751 951
- FR-A- 948 326
- FR-A- 2 352 776
- US-A- 4 573 995
- DATABASE WPI Section Ch, Week 8822 Derwent Publications Ltd., London, GB; Class A12, AN 88-152039 & JP-A-63 093 714 (SEKISUI CHEM IND KK) , 25 April 1988 & PATENT ABSTRACTS OF JAPAN vol. 12 no. 333 (C-526) ,8 September 1988 & JP-A-63 093714 (SEKISUI CHEM CO LTD) 25 April 1988,
- CHEMICAL ABSTRACTS, vol. 108, no. 14, 4 April 1988 Columbus, Ohio, US; abstract no. 118822, MATYNIA A. ET AL 'Quality of ascorbic acid crystals obtained from aqueous solutions' & PRZEM. CHEM., vol. 66, no. 6, 1987 pages 288-289, MATYNIA A. ET AL 'Quality of ascorbic acid crystals obtained from aqueous solutions'
- Bulletin Officiel de la Propriete Industrielle 2.209.544, published 7 December 1973 (07.12.73) (Syntex USA Inc.) page 11868.
- Dorland's Illustrated Medical Dictionary,1985,"cream","ointment"

## Description

The present invention relates to compositions incorporating biologically active agents and to methods of preparing such compositions. The invention also provides methods of treatment using such compositions.

It has been postulated that the efficacy of many active agents would be increased if those agent could be incorporated into a composition that is suitable for topical application and subsequently be delievered transdermally. It is believed that, for example, in the case of anti-inflammation and analgesics, the efficacy of the agent would increase because the agent is being delivered directly to the site of the inflammation or pain. Furthermore, delivery of the agent by topical application may avoid the undesirable side effects many active agents have on the gastro-intestinal tract (e.g. stomach bleeding, ulceration) and decrease or loss of activity caused by metabolic decomposition of the active agent.

Australian patent application number 33006/89 by Medvet Science Pty Ltd describes a topical anti-inflammatory or anti-irritant composition which includes a zinc salt of one or more compounds selected from unsaturated fatty acids, polyunsaturated fatty acids and cyclic derivatives thereof and aspirin in a pharmaceutically acceptable vehicle. The zinc salts used in this composition are described as being generally crystalline solids or greasy pastes (depending upon the degree of saturation) and should be stored at -70°C in a CO₂ or N₂ atmosphere for optimum stability.

Suitable vehicles/carriers for the composition described in AU-A-33006/89 include ethanol, dimethylsulphoxide (DMSO), isopropanol, glycerol, propylene glycol, dimethylacetamide and mixtures thereof. The compositions are formulated by dissolving or dispersing the dry zinc salt in DMSO and subsequently diluting with glycerol. AU-A-33006/89 does not describe how the aspirin is incorporated into the composition.

United States patent no. 4555524 by Gruber et al describes a transdermal medicament for the delivery of 2-(4-isobutylphenyl)-propionic acid (commonly known as ibuprofen) to a patient. The medicament comprises a base capable of dissolving ibuprofen, which base contains 30-50 parts by weight C₆₋₁₂ carboxylic acid triglyceride, 4-10 parts by weight glycerol monostearate - polyoxyethylene stearate mixture and 2-10 parts by weight polyoxyethylene fatty acid ester. According to Gruber et al, it is not possible to prepare an ibuprofen cream by conventional cream preparation methods because ibuprofen is only sparingly soluble in water and conventional media. Previous attempts to produce an ibuprofen cream have resulted in a product in which the ibuprofen was present in the form of fine, acicular crystals. Our tests on current commercially available ibuprofen powder have shown that the majority of the ibuprofen crystals in the powder have a largest dimension of 60 microns or greater. If these crystals are simply compounded with conventional cream bases, it is likely that the particle size of the ibuprofen crystals would remain substantially unaltered. Gruber et al have attempted to overcome the problem by using a fairly complex, non-conventional base that is capable of dissolving ibuprofen.

A paper by Patel, U. and Banakar, U.V. entitled "Comparative In-Vitro Dermatokinetics of Ibuprofen," Il Farmaco, vol 45, no 5, 1990, pp 559-568, discussed the formulation of a number of ibuprofen dermal systems. Several ibuprofen ointments were prepared. However, Patel and Banakar concluded that the formulation may not be able to provide for an immediate effect/relief by releasing an adequate amount at the desired site. Patel and Banakar do not describe the method they use to produce the ibuprofen ointments, but it appears that they simply mixed the ibuprofen with conventional ointment bases.

Patel and Banakar suggest (at page 65) that potentially higher dermal doses of IBP (Ibuprofen) could be achieved by using a water-in-oil system composition (Cold Cream). However, the paper provides no evidence to support this assertion.

United States patent no. 4511563 to Schmolka discloses analgesic gels that include 5-15 parts of an analgesic compound, 10-40 parts of a non-ionic surfactant, 5-40 parts glycerin and 40-70 parts water. The surfactants are essential to produce the required product in the form of a gel.

United States patent no. 4665063 by Bar-Shalom describes a method for treating skin disorders by applying a composition comprising acetyl salicyclic acid and a suitable carrier. The patent does not describe how the compositions are manufactured; the only requirement being that the acetyl salicyclic acid is dissolved in the carrier.

Ascorbic acid is a biologically active agent which has properties which make the formulation of compositions for transdermal delivery extremely difficult.

Ascorbic acid is notoriously unstable and although it is soluble in water its propensity to oxidise in aqueous solution and/or degrade due to attack by water itself make it desirable to minimise the use of water. On the other hand, the solubility of ascorbic acid in non-aqueous solvents is relatively poor. Consequently despite numerous attempts to produce stable compositions for transdermal delivery concentrations of ascorbic acid in commercially useful formulations have generally been limited to less than 10% ascorbic acid.

U.S. Patent 4,983,382 and International Patent Application PCT/US90/01968 describe compositions comprising higher concentration of ascorbic acid using water and a co-solvent such as an alcohol or glycol. However these inventions rely on presence of water to solubilise sufficient ascorbic acid to provide an effective composition.

EP-A-0 156 080 describes a conformable percutaneous absorption device for delivering a drug by absorption through the skin or mucosa.

EP-A-0 151 953 describes a pharmaceutical composition for topical administration consisting of two liquid phases designed to be admixed in situ or prior to use.

WO 92/08445 describes a palatable liquid therapeutic microemulsion wherein a drug dissolved in propylene glycol is dispersed in fatty ester.

US 4,156,736 describes the production of supersaturated infusion solutions of isosorbide dinitrate.

Whilst there have been many formulations for the transdermal delivery of biologically active agents in the past, in many cases, the properties of the biologically active agents have been such that it has been difficult to formulate them in such a way as to give compositions which are stable and are able to give an adequate and sustained rate of release of agent to the body when applied directly to the skin.

In particular the degree of solubility in the carrier has often been a limiting factor.

The applicants have now found that the rate of transdermal delivery of biologically active compounds present in a carrier at a level above their solubility limit in the carrier, when applied directly to the skin, can be increased and controlled by having at least a substantial proportion of the active compounds present in the carrier in the form of fine particles.

According to a first aspect the invention provides a composition for transdermal administration of a biologically active agent comprising said biologically active agent and a pharmaceutically acceptable carrier wherein said pharmaceutically acceptable carrier comprises at least one liquid that does not undergo significant reaction with the biologically active agent, wherein the biologically active agent is present at a concentration above its solubility limit in said carrier at ambient conditions and wherein there are sufficient fine particles of said agent dispersed through said carrier to substantially facilitate the transdermal transfer capacity of said composition.

As hereinafter used throughout this specification, the term "biologically active agent" shall be taken to include pharmaceuticals, therapeutic agents and prophylactic agents.

It will be appreciated that the composition may include a single biologically active agent or two or more compatible biologically active agents. It is therefore to be understood that the term "biologically active agent" also encompasses two or more biologically active agents.

Said biologically active agent will be present as a saturated or super saturated solution in said carrier. However, at least 60% of the solid particles of active agent are sized less than 20 microns. More preferably, at least 30% of the solid particles are sized less than 10 microns. Even more preferably, at least 60% of the particles are sized less than 10 microns. In a most preferred embodiment, at least 30%, even more preferably at least 60% of the particles are sized less than 5 microns.

The effective rate of transfer will be dependent on the desired delivery rate and dosage required for the particular biologically active agent concerned. However, in many instances the compositions would be formulated in such a way as to ensure that at least 30% of the biologically active agent present in the composition would be delivered transdermally within 150 hours, preferably 48 hours, more preferably 3 hours of direct application of the composition to the skin.

It is even envisaged that there be a range of preferred particle sizes to give a pre-planned dosage rate over a defined period. A portion, for example, 5 to 80% of the particles, may be extremely fine, for example less than 5 microns, and preferably in the range of 2 to 5 microns, in order to give a very high imediate range of transdermal effect. A second portion of the particles (for example 5 to 94%) may be of a larger size, for example less than 20 microns and preferably in the range of from 5 to 20 microns, and a third portion, say 1 to 40%, may be larger than 20 microns, to ensure an ongoining slow increase of activity over a period. Alternatively, a portion of the particles may be encapsulated in an outer layer which is adapted to delay the activity of the encapsulated particles until the outer layer dissolves or is in some other way dissipated by any means such as melting by body heat being dissolved by fluids on skin or in a second component added before applcation etc.

The compositions may include stabilisers to maintain stability at normal storage temperatures particularly room temperature, or they may be formulated in such a way as to ensure stability at such temperature.

The composition may further comprise one or more of a stabiliser, a dispersing agent, an emulsifier, a thickening agent and other ointment bases.

In another embodiment the size of said particles is graded to ensure an effective rate of transdermal release over an extended period of time.

In one embodiment said period is a least 3 hours.

In another embodiment said period is at least 48 hours.

In another embodiment said period is at least 150 hours.

In another aspect this invention comprises formulating an active agent, preferably ibuprofen, aspirin, captopril or ascorbic acid, in a carrier so as to provide a saturated solution of the said active agent and wherein at least 60% of the particulate active agent is present in the form of solid particles sized less than 20 microns.

For the purposes of this specification the particle size of the particles was determined by microscopic examination. It should be assumed that the particles are of a constant thickness. The surface area of each visible particle face should be determined, and the particle size calculated by calculating the square root of the visible surface area of each particle. This value should then be taken to the average length of side and hence size of that particle.

The biologically active agent used in the composition is advantageously a pharmaceutically active agent. Examples of suitable pharmaceutically active agents include aspirin, 2-(4-isobutylphenyl)-propionic acid (ibuprofen), ascorbic acid and captopril. It will be appreciated that the above list is not exhaustive and that the invention also encompasses the use of pharmaceutically active agents other than those specifically mentioned.

Other biologically active agents that may be incorporated into the composition according to the present invention include but are not limited to the following:
Non-steroidal anti-inflammatory agents, for example, aspirin, salicylates, indomethacin, aryl and hyteroaryl alkonic acids, ibuprofen, ketoprofen and flubiprofen;
Anti-asthmatic agents, for example, theophyline;
Motion sickness agents, for example, scopolamine;
Dermatological disorder drugs, for example, retinoids, antibiotics, mysteclin, minomycin;
Opiates, for example, morphine, methadone and all pain relieving compounds, such as codeine;
Narcotic agonists and antagonists, for example, naloxone;
Anticonvulsants, for example, phenytoin, carbamazepine. (Absorption of such drugs by the gastro-intestinal tract is frequently erratic);
Sedatives/hypnotics, for example, benzodiazepines;
Local anaesthetics, for example, lidocaine;
Peptides, for example growth factors, insulin;
Streptokinase;
Antiviral drugs, for example, AZT;
Contraceptives;
Calcium channel blockers, for example, nifedipine;
Anti-hypertensive drugs, for example, prazosin, propranolol, guanethidine, clonidine; and
ACE inhibitors, for example, captopril.

Examples of pharmaceutically acceptable carriers include those selected from the group consisting of water, polyhydric alcohols including alkylene glycols, (particularly propylene glycol) and glycerol; alcohols such as ethanol and isopropanol; polyalkylene glycols such as polyethylene glycol and polypropylene glycol; other ointment bases such as petroleum jelly, lanolin, dimethylformamide, ethylene glycol, tetrahydrofurfuryl alcohol, cyclohexane, cyclohexanone, acetone, ethylether, N-dodecylazocyclo-heptan-2-one, methyldecylsulfoxide, dimethylacetamide and diethylfoluamide; and mixtures thereof.

In a preferred embodiment wherein the biologically active agent is ascorbic acid, said ascorbic acid is present in an amount of from 20 to 45% by weight of the composition and the pharmaceutically acceptable carrier is selected from the group consisting of glycerol, propylene glycol, polyethylene glycol.

In another embodiment wherein the biologically active agent is a non-steroidal anti-inflammatory agent said composition further includes aspirin or ascorbic acid or mixtures thereof.

In another embodiment wherein the biologically active agent is ibuprofen said ibuprofen is present in an amount of from 15 to 35% by weight of the composition.

the preferred carriers are inert liquids, that is, they do not undergo any significant reaction with the active agent.

It should be appreciated that the above list is not exclusive as the present invention also encompasses the use of pharmaceutically acceptable carriers other than those specifically mentioned. Glycerol, propylene glycol and polyethylene glycol are preferred and glycerol has been found to be particularly useful. Where the active agent is ascorbic acid then preferably the carrier contains little or no water and preferably contains less than about 0.5% by weight water.

Compositions according to the present invention have been found to be suitable for supplying or delivering a biologically active agent for therapeutic and/or prophylactic purposes in both human and veterinary applications.

In one embodiment the invention involves use of the hereinbefore defined composition in treatment of a human or animal disorder.

Accordingly, in a further aspect, the invention provides a method for delivering a biologically active agent transdermally to a body which comprises applying a composition according to the invention to the skin of the body. In a particular aspect the present invention includes a method for the treatment and/or preventoin of a disorder in a human or animal which comprises administering an effective amount of a composition described herein to the human or animal.

In a particularly preferred embodiment the invention involves use of a composition as hereinbefore defined in treatment of a localised inflammation wherein said active agent is an anti-inflammatory such as ibuprofen.

Compositions of the present invention have been found to be particularly efficacious in delivering the biologically active agent by transdermal migration following topical application, and this is the preferred route for administering the compositions. The compositoins may also be administered orally or by parenteral or subdermal deposition.

The present invention also includes methods for producing the compositions described herein.

Accordingly, in yet a further aspect, the present invention includes a method for producing a composition, which composition comprises a pharmaceutically acceptable carrier and a biologically active agent, which method comprises:
(a) dissolving an amount of said biologically active agent in the pharmaceutically acceptable carrier at an elevated temperature, said amount of biologically active agent being in excess of the solubility limit in the carrier at ambient conditions; and
(b) cooling the mixture under conditions such that sufficient of the particles of biologically active agent that form are sufficiently fine to facilitate transdermal transfer of said biologically active agent when said composition is topically applied.

Preferably, any crystals of biologically active agent that form are predominantly sized less than 20µm.

The method of the present invention may be carried out by heating the pharmaceutically acceptable carrier to the desired temperature and then adding the biologically active agent.

Alternatively, a mixture of the organic active agent and the carrier may be produced at room temperature and subsequently heated to dissolve substantially all of the biologically active agent. Preferably, the mixture is stirred whilst it is being heated.

The mixture may be heated by conventional means or by microwave radiation, with microwave heating being preferred.

In the case of ascorbic acid, ibuprofen, aspirin and captopril the mixture may be heated to a temperature in the range of from 60 to 170°C and more preferably 100 to 130°C.

For biologically active agents, in general the minimum temperature required to obtain complete dissolution of the particles is preferably used, particularly in the case of active agents and/or carriers which are liable to decompose at excessive temperatures.

It will be appreciated that the temperature required to be used will vary according to the solubility of the biologically active agent in the carrier and the degradation temperature of the biologically active agent.

The cooling step of the present invention may be used to control the particle size of any particles of biologically active agent that form. The cooling step may involve rapidly cooling the heated mixture to ambient or below ambient temperature. This quick cooling (or "shock cooling") of the mixture can result in the formation of small particles of biologically active agent. Large particles generally do not grow because of rapid cooling and resultant increase in viscosity largely prevents diffusion of biologically active agent in solution to the particles that may have nucleated.

Quick cooling may be achieved by placing the mixture in an ice bath.

An alternative cooling step involves allowing the mixture to slowly cool. This cooling method requires that the mixture be viscous. The high viscosity of the mixture will largely prevent diffusion or reduce the rate of diffusion of dissolved biologically active agent to any particles of biologically active agent that may nucleate. The result is particles of biologically active agent that are predominantly sized less than 20 micrometres. The viscosity of the mixture may be increased by adding viscosity modifying agents to the mixture. The pharmaceutically acceptable carrier may constitute the viscosity modifying agent. Exmaples of suitable viscosity modifying agents include polyethylene glycol and ointments based thereon, petroleum jelly and paraffin wax. In an alternative method, the composition of the

present invention is produced by heating the carrier and the biologically active agent to melt or liquefy the active agent.

The resulting liquid (or, in some cases, immiscible liquids) are then stirred to effectively disperse the molten active agent throughout the carrier. The mixture is then cooled whilst maintaining stirring to produce a composition according to the present invention.

Thus, it will be appreciated that the temperature to which the mixture is heated will be governed by:
(i) the lowest temperature required to obtain a liquid phase or liquid phases (whether by dissolution of the active agent in the carrier or by melting of the active agent), and
(ii) the degradation temperature of the active agent and/or carrier.

Clearly, if the temperature required to produce one or more liquid phases is above the degradation temperature of either the active agent or the carrier, another method of preparation will have to be used. For example, particles of biologically active agents in the chosen sizes may be prepared, (for example by grinding) and subsequently mixed with a pharmaceutically acceptable carrier to produce a composition in accordance with the invention.

The invention will now be described with reference to the following examples.

### EXAMPLE 1

A composition containing ibuprofen was prepared by adding 20.6g ibuprofen to a heated 130°C solution consisting of 26.2 g glycerol, 21.6g propylene glycol and 2.5g polyethylene glycol (20000). The solution was stirred at this temperature until it became clear and then transferred into a vessel controlled at 4°C in which it was stirred until a creamy paste formed. The paste was allowed to remain at this low temperature for a further 15 minutes and then stirred again to form a viscous paste.

The particle size of the ibuprofen crystallites precipitated during the cooling and stirring of the above formulation was compared with that of the unreacted ibuprofen powder. The respective particle sizes were determined by microscopic examination. It was assumed that the crystals were of a constant thickness. The surface area of each crystal was then determined, and the particle size calculated by calculating the square root of the measured surface area. This value was then taken to be the length of side. The total area of the counted particles was measured and the sum of the areas of the different size ranges estimated as a percentage of the total. The results were:

**Table 1**

| **Ibuprofen/Glycerol Composition of the Present Invention** | |
|---|---|
| Size Range | Percent of Particles in Size Range |
| <2µm | 0.35 |
| 2-5µm | 32.4 |
| 5-10µm | 42 |
| 10-15µm | 23 |

These particle sizes are much smaller than the particle sizes of ibuprofen in a commercially available cream.

### EXAMPLE 2

This example demonstrates the preparation of a composition containing Ibuprofen.

40 parts by weight of Ibuprofen is added to 93 parts by weight of propylene glycol heated to 130°C. The solution was stirred until clear and then quickly cooled under solidified carbon dioxide with stirring. This produces a soft white paste.

### EXAMPLE 3

20.6g of Ibuprofen was dissolved in 45 g propylene glycol heated to between 120 to 130°C. A clear solution was obtained. This solution was stirred while cooling to obtain a creamy white formulation with small (less than 10 microns) ibuprofen particles.

### EXAMPLE 4

20.6 g Ibuprofen was dissolved in 55g propylene glycol at 120°C. The resulting clear solution was added to a jacketed container surrounded by water at 6°C. Upon stirring, a creamy white formulation with small particles (less than 10 microns) of ibuprofen was formed.

### EXAMPLE 5

30g Ibuprofen was dissolved in a heated mixture containing 40g propylene glycol and 30g glycerol. The clear solution was allowed to cool and was shaken for 5 minutes to give a creamy white material with particles of ibuprofen less than 10 microns.

Part of this was reheated by microwave heating to form a clear solution and the rapidly chilled to around 0 C. The thick clear gel was then stirred and again a white cream formed with ibuprofen particles of 2-5 microns.

### EXAMPLE 6

30g Ibuprofen was added to 70g glycerol heated to 120 C. The ibuprofen melted and formed an immiscible top layer. The mixture was stirred vigorously while cooling to give a white cream. Small crystals of ibuprofen (less than 5 microns) were present, but aggregates of the small crystals were also formed.

The sample was ground by a mortar and pestle to remove the aggregates. The transdermal transfer of this formulation was tested in a Franz cell and compared against a commercially available ibuprofen cream. After 22 hours, the compositon according to the example had delivered 306 micrograms of the ibuprofen across the skin membrane for the Franz cell. In the same time, the commercially available ibuprofen cream delivered 170 microgroms ibuprofen.

Compositions of ibuprofen in glycerol, according to the present invention, exhibit an abilty to alleviate pain with topical application. The rebound of pain after about 3-4 hours indicates an analgesic effect resulting possibly from the anti-inflammatory action of the ibuprofen. The greater the concentration of ibuprofen represented by the number of the very small crystallites the greater the effect.

### EXAMPLE 7

This example demonstrates the preparation of a composition containing aspirin.

Add 21.7 parts by weight aspirin to a solution containing 24.9 parts by weight glycerol, 20.6 parts by weight propylene glycol and 5 parts by weight polyethylene glycol (20000). The solution is kept at under 95°C and stirred until all aspirin is dissolved. The solution is rapidly cooled, e.g. by quenching with dry ice, while stirring. This produces a white paste which shows only a slight salicylic acid reaction with Fe³⁺ solution (i.e. the aspirin is not broken down to salicylic acid).

### EXAMPLE 8

A composition containing ascorbic acid was prepared by the method of the invention. 35.2g ascorbic acid was dissolved in a heated solution containing 43.2g propylene glycol and 5g polyethylene glycol (20000) at 140°C. The solution was cooled to about 100°C and then rapidly cooled in a jacketed vessel to 4°C with stirring, until the material became a very viscous cream. It was bottled and a sample examined by optical microscopy.

For comparative purposes, unformulated crystals of commercially available ascorbic acid were examined. In the simple mixing of the crystals with a carrier, it is expected that the particle size of the crystals would not be markedly altered.

Both the comparative mixture and the composition produced according to the invention were examined under a microscope. The comparative mixture had ascorbic acid crystals that were approximately square shaped (equidimensional). The ascorbic acid crystals of the composition of the invention contained crystals of varied shape, with equidimensional crystals being the most common.

The particle size of the crystals were determined by microscopic examination. It was assumed that the crystals were of a constant thickness. The surface area of each crystal was then determined, and the particle size calculated by calculating the square root of the measured surface area. This value was then taken to be the length of side. The total area of the counted particles was measured and the sum of the areas of the different size ranges estimated as a percentage of the total. The results were:

**Table 1**

| **Ascorbic Acid - Carrier Composition According to the Present Invention** | |
|---|---|
| Size range. | Percentage Particles in Size range |
| < 2µm | 12.7 |
| 2-5µm | 42.5 |
| 5-10µm | 45.8 |

**Table 2**

| **Unformulated Ascorbic Acid: Comparative Example** | |
|---|---|
| Size range. | Percentage Particles in Size range |
| 10-20µm | 0.36 |
| 20-50µm | 0.57 |
| 50-100µm | 2.7 |
| >100µm | 96 |

As can be seen by comparing the data in Tables 1 and 2, the composition of the present invention has ascorbic acid particles with a much smaller particle size than that of the comparative example.

Tests using radioactive labelled ascorbic acid have shown that the ascorbic acid, when formulated according to the method of the present invention, is capable of migrating transdermally and becoming biologically available.

### EXAMPLE 9

40g ascorbic acid was dissolved in 60g glycerol heated to 130°C. The resultant pale yellow solution was sitrred with cooling. As the temperature dropped the mixture became a viscous opaque cream as small particles of ascorbic acid came out of solution.

The stirring was discontinued and the cream allowed to stand at room temperature and became an off-white waxy material.

### EXAMPLE 10

Two compositions comprising ascorbic acid and glycerol were produced. Composition A was produced by adding 3 parts ascorbic acid by weight to 7 parts glycerol by weight. The mixture was heated to 120-130°C to dissolve the ascorbic acid. The heated mixture was quenched by adding dry ice. Ascorbic acid particles present in composition A were predominantly in the size range of 2-10 microns.

Composition B was prepared by mixing C¹⁴ labelled ascorbic acid with unlabelled ascorbic acid. The ascorbic acid was dissolved and recrystallised to ensure adequate mixing of the labelled and unlabelled ascorbic acid. The recrystallised ascorbic acid was broken up on a mortar and pestle and sieved through a nylon sieve. Particles in the range of 50-100 microns were collected and mixed with glycerol in a weight ratio of 3 to 7. No attempt was made to heat this mixture.

Composition A was applied to two laboratory rats and ascorbic acid levels in blood and uring were measured over a six hour period and the mean of the two rats determined at each time period.

The same tests were conducted on two other laboratory rats using composition B. The results were plotted to show the variation of ascorbic acid match and are shown in the attached drawings.

In the drawings:
Figure 1 is a graph showing the variation of ascorbic acid concentration in urine in the period of 6 hours of the topical application of composition A (size 2-10 microns) and composition B (50-100 microns).
Figure 2 is a graph showing the variation of blood plasma ascorbic acid concentration in the period of 5 hours of the topical application of composition A (size 2-10 microns) and composition B (50-100 microns).
Figure 3 is a graph showing the variation over 22 hours of ibuprofen and is referred to in Example 6. The plot marked "FINE" indicates results in administration of a formulation containing ibuprofen of particle size 5 micrometres. The plot marked "COARSE" indicates results using an equivalent formulation in which the particles were essentially greater than 50 micrometres.

### EXAMPLE 11

35.2g ascorbic acid was added to a ternary mixture containing 43.2g propylene glycol, 52.4g glycerol and 5g polyethylene glycol heated to 140°C. The solution was cooled to about 100°C and then added to a jacketed vessel cooled with water at 4°C. The solution was stirred with a propeller stirrer until it became too viscous, after which the stirrer was raised. The composition adhering to the stirrer bladed showed some very small crystals. The product was kept chilled in the jacketted vessel for 1.5 hours before storing at room temperature.

### EXAMPLE 12

40g ascorbic acid was dissolved in 80g heated polypropylene glycol and this was then added to a cooled jacketted vessel at 4°C. A whitish paste formed and was shown to consist predominantly of 2-5 micrometre sized particles of ascorbic acid.

### EXAMPLE 13

### IBUPROFEN (BRUFEN^{®})

A comparison of the transdermal permeation of fine and coarse ibuprofen was made using skins from shaved Hooded Wistar rats as membranes in 2 side-by-side Franz in vitro cells. Reference Percutaneous absorption. On the relevance of in vitro data. J. Investigatige Dermatology, Vol 64, 1975, 190-195, T.J. FRANZ). Tritium labelled ibuprofen was used. The fine particles (essentially < 5 microns) were formulated in glycerol; 3 part ibuprofen to 7 part glycerol w/w) according to the method of the invention. The coarse material (essentially > 50 microns) was mixed with glycerol in the same proportions.

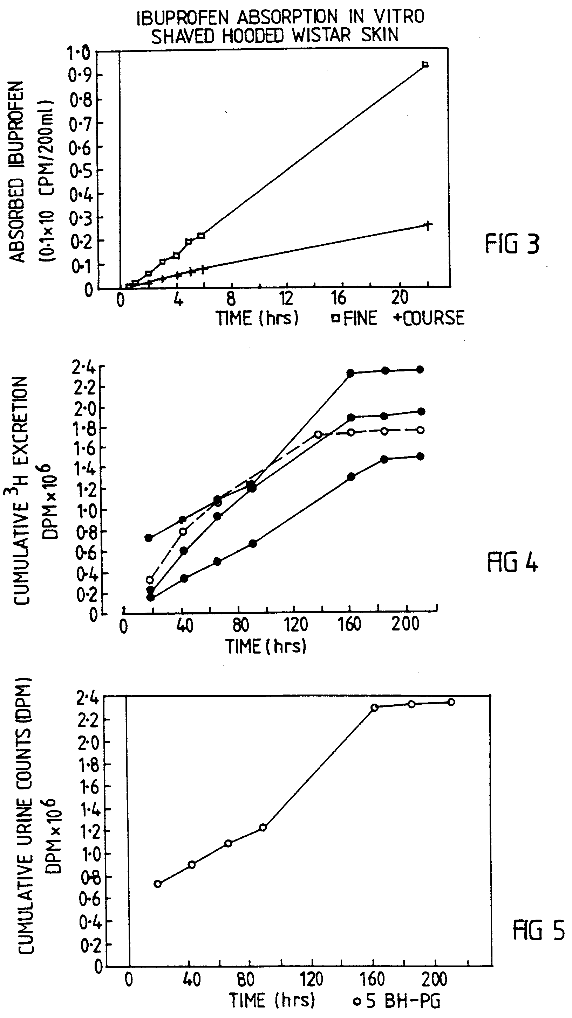

Figure 3 shows that the variation over 22 hours in the permeation of the two size ranges of ibuprofen through the two skin sections following the application of approximately equal amounts of the respective formulations to similar areas.

### EXAMPLE 14

Four anesthetized Hooded Wistar rats were fitted with jugular and bladder catheters. The dorsal skin of HOODED WISTAR RATS was shaved and a composition according to the present invention containing ibuprofen was topically applied to the shaved skin. Plastic covers were placed over the shaved area to prevent the animals ingesting the applied composition.

The animals were placed in metabolism cages and the urinary ibuprofin monitored.

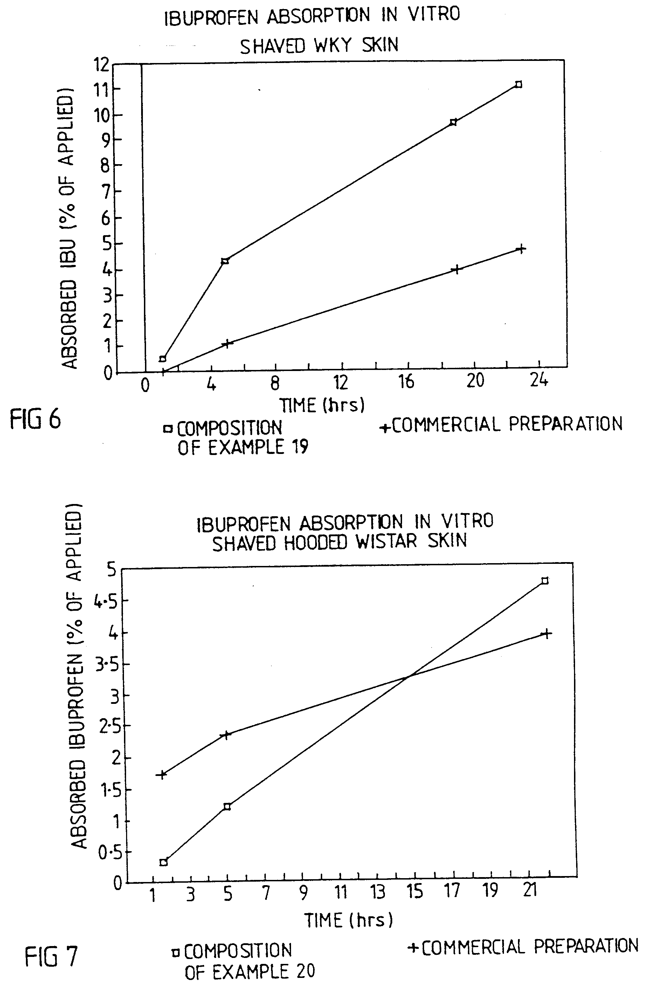

The amount of ibuprofen detected in each of the rats is shown in Figure 4.

The results show that the absorption of ibuprofen has zero order kinetics with an extremely long half life of from 80 to 100 hours and was still being excreted more than 160 hours after topical application. In contrast, oral and IV administered ibuprofen has a half life of the order of 30 minutes.

### EXAMPLE 15

A rat was anesthetized and clipped and shaved in the dorsal area just below the neck. Labelled ibuprofen in polypropylene glycol was applied to the shaved area. The area of application was then covered witha plastic cover. The amount of ibuprofen excreted in the urine was determined and the results shown in Figure 5.

### EXAMPLE 16

The method of Example 6 was repeated using ¹⁴C labelled ascorbic acid of corresponding particle sizes.

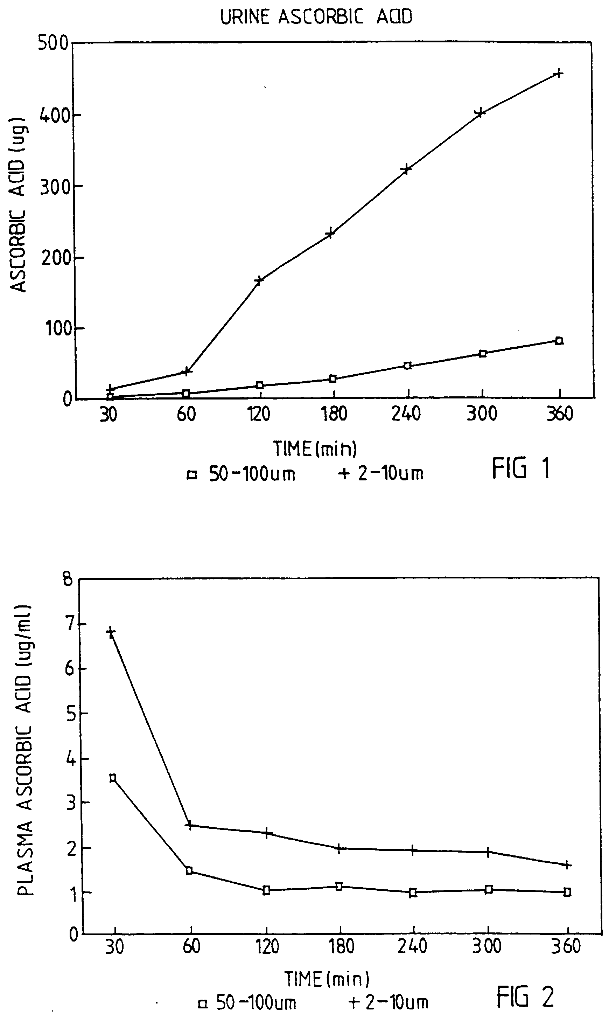

Figure 2 shows the variation over six hours in permeation of the two size ranges of ascorbic acid through the two skin sections following application of approximately equal amounts of respective formulations to similar areas.

### EXAMPLE 17

A mixture containing 18.24g propylene glycol, 22.1g glycerol and 10.08g polyethylene glycol (20000) was heated to 100°C. 10g ascorbic acid and 10g aspirin were added and stirred until dissolved. Dry ice was added to the solution, which was stirred until cold. A white viscous cream was obtained.

### EXAMPLE 18

Captopril was dissovled in heated glycerol and cooled in the refrigerator. After cooling to 4° C, the solution which was stirred and a white cream contining particles sized predominantly less than 5 microns was obtained.

### EXAMPLE 19

A composition was prepared by adding 30g ibuprofen to a heated mixture containing 40g propylene glycol and 30g glycerol. The ibuprofen dissolved in the propylene glycol. The solution was cooled slightly and divided into two parts. One part was shaken by hand, the other part was shaken by mixer. A white creamy material formed in both. The machine shaken part had very small particles of ibuprofen. The other sample was transferred to a beaker and reheated to give a clear solution. The beaker was placed in a sub-zero environment where the solution became viscous but remained clear. The paste was then stirred until a white creamy thin paste formed. This contained very fine particles (essentially less than 5 microns). This composition was then compared to a commercially available ibuprofen cream in Franz cells using rat skin pre-wetted with glycerol. The results from the Franz cell are shown in Figure 6. As can be seen, the composition according to the present invention is cleraly superior to the commercially available cream.

### EXAMPLE 20

A composition was prepared by adding 30g ibuprofen to 70g heated glycerol. The ibuprofen melted and formed a separate liquid layer on top of the glycerol. The mixture was stirred and subjected to ultrasonics while cooling. Some creamy material formed, but this contained many hard aggregates. Microscopic examination showed that the un-agglomerated particles were small. A sub-sample was ground in a mortar until a consistent, non-particulate cream was formed. 21.6mg of this material was applied to the skin of a Franz cell. 44.6mg of a commercially available ibuprofen cream was applied to another Franz cell. The results are shown in Figure 7. As can be seen, the commercially available cream had an initially higher rate of transdermal release, but there was a cross-over at about 15 hours, after which the composition according to the present invention was clearly superior.

Without wishing to be bound by theory, the results of Examples 19 and 20 have led the inventors to postulate that the transdermal transfer characteristics of the inventive conditions may be improved when a carrier that at least partially dissolves the biologically active agent is used. Comparing the transdermal transfer characteristics of the compositions of the invention from Examples 19 and 20, it can be shown that the composition of Example 19, which contained propylene glycol (in which ibuprofen does dissolve to a certain extent) and superior transdermal transfer characteristics to the composition of Example 20, which used glycerol as a carrier (ibuprofen being substantially insoluble in glycerol). However, both compositions showed transdermal transfer of the active agent.

## Claims

1. A transdermal composition in the form of a cream or an ointment for administration of a biologically active agent comprising a biologically active agent and a pharmaceutically acceptable carrier wherein said biologically active agent is present at a concentration above its solubility limit in said carrier at ambient conditions and wherein the biologically active agent is present in the form of fine solid particles dispersed throughout the carrier and at least 60% of the solid particles are sized less than 20 microns.

2. A transdermal composition according to claim 1, wherein at least 30% of the solid particles of active agent are sized less than 10 microns.

3. A transdermal composition according to claim 1 wherein at least 30% of the solid particles of active agent are sized less than 5 microns.

4. A transdermal composition according to claim 1, wherein at least 60% of the solid particles of active agent are sized less than 10 microns.

5. A transdermal composition according to claim 1, wherein at least 60% of the solid particles of active agent are sized less than 5 microns.

6. A transdermal composition according to any one of the preceding claims wherein said pharmaceutically acceptable carrier comprises at least one liquid that does not undergo significant reaction with the biologically active agent.

7. A transdermal composition according to any one of the preceding claims wherein said pharmaceutically acceptable carrier is selected from glycerol, propylene glycol, polypropylene glycol, other polyhydric alcohols, petroleum jelly, lanoline, dimethyformamide, ethylene glycol, tetrahydrofurfuryl alcohol, cyclohexane, cyclohexanone, acetone, ethylether, N-dodecylazocyclo- heptane-2-one, methyldecylsulfoxide, dimethylacetamide and diethylfoluamide; and mixtures thereof.

8. A transdermal composition as claimed in any preceding claim wherein the biologically active agent is selected from ascorbic acid, non-steroidal anti-inflammatory agents, anti-asthmatic agents, motion-sickness agents, drugs used in the treatment of dermatological disorders, opiates or pain relieving drugs, narcotic agonists, narcotic antagonists, anticonvulsants, sedatives, hypnotic agents, local anaesthetics, growth factors, insulin, streptokinase, peptides, anti-viral agents, AZT, contraceptives, calcium channel blockers, anti-hypertensive drugs, prazosin, propranolol, guanethidine, clonidine, ACE inhibitors; and captopril.

9. A transdermal composition according to claim 8, wherein said biologically active agent is ascorbic acid and said ascorbic acid is present in an amount of from 20 to 45% by weight of the composition and the pharmaceutically acceptable carrier is selected from glycerol, propylene glycol and polyethylene glycol and mixtures thereof.

10. A transdermal composition according to claim 8, wherein said biologically active agent is a non-steroidal anti-inflammatory agent selected from aspirin, salicylates, indomethacin, aryl and heteroaryl alkanoic acids, ibuprofen, ketoprofen and flubiprofen.

11. A transdermal composition according to claim 10, wherein said composition further includes ascorbic acid.

12. A transdermal composition according to claim 10 or claim 11, wherein the biologically active agent is ibuprofen present in an amount of from 15 to 35% by weight of the composition.

13. A transdermal composition according to claim 8, wherein said anti-asthmatic agent is theophylline.

14. A transdermal composition according to claim 8, wherein said motion-sickness agent is scopolamine.

15. A transdermal composition according to claim 8, wherein said biologically active agent used in the treatment of dermatological disorders is selected from retenoids, mystedin and minomycin.

16. A transdermal composition according to claim 8, wherein said opiate or pain relieving drug is selected from morphine, methadone or codeine.

17. A transdermal composition according to claim 8, wherein said biologically active agent is benzodiazepines.

18. A transdermal composition according to any one of the preceding claims, wherein said composition includes two or more biologically active agents.

19. A transdermal composition according to any one of the preceding claims, wherein said composition further comprises one or more of a stabilizer, a dispersing agent, an emulsifier, a thickening agent and other ointment bases.

20. A transdermal composition according to any one of the preceding claims, wherein the size of said particles is graded to ensure an effective rate of transdermal release over an extended period of time.

21. A transdermal composition according to claim 20, wherein said period is a least 3 hours.

22. A transdermal composition according to claim 20, wherein said period is at least 48 hours.

23. A transdermal composition according to claim 20, wherein said period is at least 150 hours.

24. A transdermal composition according to any one of claims 20 to 23, wherein at least 30% of the biologically active agent is delivered within said period.

25. A transdermal composition according to any one of the preceding claims, wherein 5% to 80% of the particles are less than 5 microns, 5% to 94% of the particles are between and 5 and 20 microns and 1% to 40% of the particles are greater than 20 microns.

26. A method for producing a composition according to any one of the preceding claims, said method comprising:
(a) dissolving an amount of said biologically active agent in the pharmaceutically acceptable carrier at an elevated temperature, said amount of biologically active agent being in excess of the solubility limit in the carrier at ambient conditions, and
(b) cooling the mixture to produce particles of biologically active agent that are sufficiently fine to facilitate an effective rate of transdermal transfer of said biologically active agent when said composition is topically applied.

## Patentansprüche

1. Transdermale Zusammensetzung in Form einer Creme oder einer Salbe zur Verabreichung eines biologisch aktiven Wirkstoffs, die einen biologisch aktiven Wirkstoff sowie einen pharmazeutisch zulässigen Träger aufweist, bei der der genannte biologisch aktive Wirkstoff in einer Konzentration oberhalb seiner Löslichkeitsgrenze in dem genannten Träger bei Umgebungsbedingungen vorliegt und bei der der biologisch aktive Wirkstoff in Form von feinen Festoffteilchen vorliegt, die im ganzen Träger dispergiert sind, und wobei wenigstens 60% der Feststoffteilchen eine Größe von weniger als 20µm aufweisen.

2. Transdermale Zusammensetzung nach Anspruch 1, bei der wenigstens 30% der Feststoffteilchen des Wirkstoffs eine Größe von weniger als 10µm aufweisen.

3. Transdermale Zusammensetzung nach Anspruch 1, bei der wenigstens 30% der Feststoffteilchen des Wirkstoffs eine Größe von weniger als 5µm aufweisen.

4. Transdermale Zusammensetzung nach Anspruch 1, bei der wenigstens 60% der Feststoffteilchen des Wirkstoffs eine Größe von weniger als 10µm aufweisen.

5. Transdermale Zusammensetzung nach Anspruch 1, bei der wenigstens 60% der Feststoffteilchen des Wirkstoffs eine Größe von weniger als 5µm aufweisen.

6. Transdermale Zusammensetzung nach irgendeinem der vorausgehenden Ansprüche, bei der der pharmazeutisch zulässige Träger wenigstens eine Flüssigkeit umfaßt, die mit dem biologisch aktiven Wirkstoff keine nennenswerte Reaktion eingeht.

7. Transdermale Zusammensetzung nach irgendeinem der vorausgehenden Ansprüche, bei der der pharmazeutisch zulässige Träger ausgewählt ist aus Glycerol, Propylenglykol, Polypropylenglykol, anderen mehrwertigen Alkoholen, Vaselin, Lanolin, Dimethylformamid, Ethylenglykol, Tetrahydrofurfurylalkohol, Cyclohexan, Aceton, Ethylether, N-Dodecylazocycloheptan-2-on, Methyldecylsulfoxid, Dimethylacetamid und Diethyltoluamid und Mischungen davon.

8. Transdermale Zusammensetzung nach irgendeinem der vorausgehenden Ansprüche, bei der der biologisch aktive Wirkstoff ausgewählt ist aus Ascorbinsäure, nicht steroidalen entzündungshemmenden Mitteln, Antiasthmamitteln, Reisekrankheitsmitteln, Arzneimitteln, die zur Behandlung von dermatologischen Erkrankungen verwendet werden, Opiaten oder schmerzlindernden Arzneimitteln, Narkotikums-Agonisten, Narkotikums-Antagonisten, Krampfmitteln, Sedativa, hypnotischen Mitteln, Lokalanästhetika, Wachstumsfaktoren, Insulin, Streptokinase, Peptiden, Antivirusmitteln, AZT, Kontrazeptiva, Calciumkanal-Blockern, blutdrucksenkenden Arzneimitteln, Prazosin, Propranolol, Guanethidin, Clonidin, ACE-Inhibitoren und Captopril.

9. Transdermale Zusammensetzung nach Anspruch 8, bei der der genannte biologisch aktive Wirkstoff Ascorbinsäure ist und die Ascorbinsäure in einer Menge von 20 bis 45 Gew.-% der Zusammensetzung vorliegt und bei der der pharmazeutisch zulässige Träger ausgewählt ist aus Glycerol, Propylenglykol und Polyethylenglykol und Mischungen davon.

10. Transdermale Zusammensetzung nach Anspruch 8, bei der der genannte biologisch aktive Wirkstoff ein nicht steroidales entzündungshemmendes Mittel ist, das ausgewählt ist aus Aspirin, Salicylaten, Indomethacin, Aryl- und Heteroarylalkansäuren, Ibuprofen, Ketoprofen und Flubiprofen.

11. Transdermale Zusammensetzung nach Anspruch 10, bei der die genannte Zusammensetzung außerdem Ascorbinsäure enthält.

12. Transdermale Zusammensetzung nach Anspruch 10 oder Anspruch 11, bei der der biologisch aktive Wirkstoff Ibuprofen ist, das in einer Menge von 15 bis 35 Gew.-% der Zusammensetzung vorliegt.

13. Transdermale Zusammensetzung nach Anspruch 8, bei der das Antiasthmamittel Theophyllin ist.

14. Transdermale Zusammensetzung nach Anspruch 8 , bei der das Reisekrankheitsmittel Scopolamin ist.

15. Transdermale Zusammensetzung nach Anspruch 8, bei der der genannte biologisch aktive Wirkstoff, der zur Behandlung von dermatologischen Erkrankungen verwendet wird, ausgewählt ist aus Retinoiden, Mystedin und Minomycin.

16. Transdermale Zusammensetzung nach Anspruch 8, bei der das genannte Opiat oder schmerzlindernde Mittel ausgewählt ist aus Morphin, Methadon oder Kodein.

17. Transdermale Zusammensetzung nach Anspruch 8, bei der der biologisch aktive Wirkstoff Benzodiazepine sind.

18. Transdermale Zusammensetzung nach irgendeinem der vorausgehenden Ansprüche, bei der die Zusammensetzung zwei oder mehr biologisch aktive Wirkstoffe enthält.

19. Transdermale Zusammensetzung nach irgendeinem der vorausgehenden Ansprüche, bei der die genannte Zusammensetzung außerdem einen oder mehrere von einem Stabilisator, einem Dispergiermittel, einem Emulgator, einem Verdickungsmittel und anderen Salbengrundlagen enthält.

20. Transdermale Zusammensetzung nach irgendeinem der vorausgehenden Ansprüche, bei der die Größe der genannten Teilchen abgestuft ist, um einen wirksamen Grad der transdermalen Freigabe über einen ausgedehnten Zeitraum sicherzustellen.

21. Transdermale Zusammensetzung nach Anspruch 20, bei der der genannte Zeitruam wenigstens drei Stunden beträgt.

22. Transdermale Zusammensetzung nach Anspruch 20, bei der der genannte Zeitraum wenigstens 48 Stunden beträgt.

23. Transdermale Zusammensetzung nach Anspruch 20, bei der der genannte Zeitraum wenigstens 150 Stunden beträgt.

24. Transdermale Zusammensetzung nach irgendeinem der Ansprüche 20 bis 23, bei der wenigstens 30% des biologisch aktiven Wirkstoffs innerhalb des genannten Zeitraums zugeführt werden.

25. Transdermale Zusammensetzung nach irgendeinem der vorausgehenden Ansprüche, bei der 5% bis 80% der Teilchen kleiner sind als 5µm, 5% bis 94% der Teilchen zwischen 5 und 20µm liegen und 1% bis 40% der Teilchen größer sind als 20µm.

26. Verfahren zur Herstellung einer Zusammensetzung nach irgendeinem der vorausgehenden Ansprüche, wobei das Verfahren umfaßt:
a) Auflösen einer Menge des genannten biologisch aktiven Wirkstoffs in dem pharmazeutisch zulässigen Träger bei einer erhöhten Temperatur, wobei die genannte Menge des biologisch aktiven Wirkstoffs höher ist als die Löslichkeitsgrenze in dem Träger bei Umgebungsbedingungen und
b) Abkühlen der Mischung, um Teilchen des biologisch aktiven Wirkstoffs zu erzeugen, die ausreichend fein sind, um einen wirksamen Grad des transdermalen Transfers des genannten biologisch aktiven Wirkstoffs zu ermöglichen, wenn die genannte Zusammensetzung topisch angewendet wird.

## Revendications

1. Composition transdermique sous forme d'une crème ou d'un onguent pour l'administration d'un agent biologiquement actif comprenant un agent biologiquement actif et un excipient pharmaceutiquement acceptable, dans laquelle l'agent biologiquement actif est présent en une concentration au-dessus de sa limite de solubilité dans ledit excipient aux conditions ambiantes et dans laquelle l'agent biologiquement actif est présent sous forme de fines particules solides dispersées dans la totalité de l'excipient et au moins 60% des particules solides ont une dimension inférieure à 20 microns.

2. Composition transdermique selon la revendication 1, dans laquelle au moins 30% des particules solides de l'agent actif ont une dimension inférieure à 10 microns.

3. Composition transdermique selon la revendication 1, dans laquelle au moins 30% des particules solides de l'agent actif ont une dimension inférieure à 5 microns.

4. Composition transdermique selon la revendication 1, dans laquelle au moins 60% des particules solides de l'agent actif ont une dimension inférieure à 10 microns.

5. Composition transdermique selon la revendication 1, dans laquelle au moins 60% des particules solides de l'agent actif ont une dimension inférieure à 5 microns.

6. Composition transdermique selon l'une quelconque des revendications précédentes, dans laquelle l'excipient pharmaceutiquement acceptable comprend au moins un liquide qui ne subit pas de réaction importante avec l'agent biologiquement actif.

7. Composition transdermique selon l'une quelconque des revendications précédentes, dans laquelle l'excipient pharmaceutiquement acceptable est choisi à partir de glycérol, propylèneglycol, polypropylèneglycol, d'autres alcools polyhydriques, vaseline, lanoline, diméthylformamide, éthylène glycol, alcool de tétrahydrofurfuryle, cyclohexane, cyclohexanone, acétone, éthyléther, N-dodecylazocyclo-heptane-2-one, méthyldécylsulfoxyde, diméthylacétamide et diéthylfoluamide et leurs mélanges.

8. Composition transdermique selon' l'une quelconque des revendications précédentes, dans laquelle l'agent biologiquement actif est choisi à partir d'acide ascorbique, d'agents anti-inflammatoires non stéroïdiens, agents anti-asthmatiques, agents contre le mal des transports, médicaments utilisés dans le traitement de troubles dermatologiques, opiacées ou médicaments soulageant la douleur, agonistes et narcotiques, antagonistes et narcotiques, sédatifs anti-convulsifs, agents hypnotiques, anesthésiants locaux, facteurs de croissance, insuline, streptokinase, peptides, agents anti-viraux, AZT, contraceptifs, inhibiteurs calciques, médicaments anti-hypertenseurs, prazosine, propanolol, guanéthidine, clonidine, inhibiteurs ACE ; et captopril.

9. Composition transdermique selon la revendication 8, dans laquelle l'agent biologiquement actif et l'acide ascorbique et l'acide ascorbique est présent dans une quantité de 20 à 45% en poids de la composition et le support pharmaceutiquement acceptable est choisi à partir de glycérol, propylène glycol et polyéthylène glycol et leurs mélanges.

10. Composition transdermique selon la revendication 8, dans laquelle l'agent biologiquement actif est un agent anti-inflammatoire non stéroïdien choisi à partir de l'aspirine, de salycilates, d'indométhacine, acide alcanoïque aryle et hétéroaryle, ibuprofène, kétoprofène et flubiprofène.

11. Composition transdermique selon la revendication 10, dans laquelle la composition comprend de plus de l'acide ascorbique.

12. Composition transdermique selon la revendication 10 ou la revendication 11, dans laquelle l'agent biologiquement actif est l'ibuprolène présent dans une quantité de 15 à 35% en poids de la composition.

13. Composition transdermique selon la revendication 8, dans laquelle l'agent anti-asthmatique est la théophylline.

14. Composition transdermique selon la revendication 8, dans laquelle l'agent contre le mal des transports est la scopolamine.

15. Composition transdermique selon la revendication 8, dans laquelle l'agent biologiquement actif utilisé dans le traitement de troubles dermatologiques est choisi à partir de réténoïdes, mystédine et minomycine.

16. Composition transdermique selon la revendication 8, dans laquelle ledit opiacé ou le médicament soulageant la douleur est choisi à partir de morphine, méthadone ou codéïne.

17. Composition transdermique selon la revendication 8, dans laquelle l'agent biologiquement actif est les benzodiazépines.

18. Composition transdermique selon l'une quelconque des revendications précédentes, dans laquelle la composition comprend deux ou plus de deux agents actifs biologiquement.

19. Composition transdermique selon l'une quelconque des revendications précédentes, dans laquelle la composition comprend de plus en plus d'un stabilisant, un agent de dispersion, un émulsifiant, un agent épaississant et l'autre base d'onguent.

20. Composition transdermique selon l'une quelconque des revendications précédentes, dans laquelle la taille des particules est choisie de façon à assurer une vitesse efficace de libération transdermique sur une durée prolongée.

21. Composition transdermique selon la revendication 20, dans laquelle ladite durée est d'au moins 3 heures.

22. Composition transdermique selon la revendication 20, dans laquelle ladite durée est d'au moins 48 heures.

23. Composition transdermique selon la revendication 20, dans laquelle ladite durée est d'au moins 150 heures.

24. Composition transdermique selon l'une quelconque des revendications 20 à 23, dans laquelle au moins 30% de l'agent biologiquement actif est délivré au cours de ladite durée.

25. Composition transdermique selon l'une quelconque des revendications précédentes, dans laquelle 5 à 80% des particules sont inférieures à 5 microns, 5 à 94% des particules se situent entre 5 et 20 microns et 1% jusqu'à 40% des particules sont supérieures à 20 microns.

26. Procédé pour la production d'une composition selon l'une quelconque des revendications précédentes, ledit procédé comprenant les étapes consistant à :
(a) dissoudre une quantité de l'agent biologiquement actif dans l'excipient pharmaceutiquement acceptable à une température élevée, ladite quantité d'agent biologiquement actif étant en excédent de la limite de solubilité dans l'excipient à des conditions ambiantes, et
(b) refroidir le mélange pour produire des particules d'agent biologiquement actif qui sont suffisamment fines pour permettre d'obtenir une vitesse efficace de transfert transdermique de l'agent biologiquement actif lorsque ladite composition est appliquée topiquement.
